# EUROPEAN PATENT SPECIFICATION

(11) **EP 1 852 135 B1**
(45) Date of publication and mention of the grant of the patent: **11.04.2018**
(21) Application number: 06714407.1
(22) Date of filing: 23.02.2006
(51) Int. Cl.: A61L 27/12, A61L 27/56, C12M 3/00

(54) **MEDICAL MATERIAL, ARTIFICIAL TOOTH ROOT AND METHOD OF PRODUCING MATERIAL FOR CLINICAL USE**
MEDIZINISCHES MATERIAL, KÜNSTLICHE ZAHNWURZEL UND VERFAHREN ZUR HERSTELLUNG EINES MATERIALS FÜR DEN KLINISCHEN EINSATZ
MATÉRIAU MÉDICAL, RACINE DE DENT ARTIFICIELLE ET PROCÉDÉ DE FABRICATION DE MATÉRIAU POUR UTILISATION CLINIQUE

(30) Priority: 23.02.2005 JP 2005047650; 23.02.2005 JP 2005047651; 25.02.2005 JP 2005052164; 16.01.2006 JP 2006008029
(43) Date of publication of application: 07.11.2007
(73) Proprietor: HI-LEX Corporation, Takarazuka-shi, Hyogo 665-0845 (JP)
(72) Inventor: SEKI, Yasuo, chome, Takarazuka-shi, Hyogo 6650845 (JP); SHIOTA, Hiroyuki, chome, Takarazuka-shi, Hyogo 6650845 (JP); KUBOKI, Yoshinori, , Hokkaido 0630038 (JP); NOISHIKI, Yasuharu, ama-shi, Kanagawa 2360005 (JP)
(74) Representative: Hancox, Jonathan Christopher
(86) International application number: PCT/JP2006/303267
(87) International publication number: WO 2006/090777

(56) References cited:
- EP-A1- 0 369 034
- EP-A1- 0 390 129
- WO-A1-01/78798
- WO-A1-03/075973
- WO-A1-2004/012781
- WO-A1-2004/084964
- FR-A1- 2 769 489
- JP-A- 63 125 260
- JP-A- 2005 001 943
- JP-A- 2005 143 800
- US-A- 5 531 794
- US-A1- 2002 104 602
- US-A1- 2005 025 807
- US-A1- 2005 025 807

## Description

### Field of the Invention

The medical material of this invention relates to an implantation material for a living body, and a structure of the shape from spherical to block etc. used for the defect portion of hard tissue or soft tissue of a tooth, a bone, and the like. The artificial tooth root of this invention presents early good connection with bones and has a periodontal membrane regeneration function having a adhesion structure similar to natural periodontal membranes. The producing method of a material for clinical use of this invention uniformly adheres the hydroxyapatite coat to the material for clinical use made of titanium.

### Background Arts

Patent Document 1: Japanese Published Patent Application NO. 2004-16398
Patent Document 2: Japanese Published Patent Application NO. 2004-67547
Patent Document 3: Japanese Published Patent Application No.H6-7381 (Japanese Patent Publication NO. H7-14400)
Patent Document 4 : Japanese Published Patent Application No.2002-159513
Patent Document 5 : Japanese Published Patent Application No.H6-47062
Patent Document 6 : Japanese Published Patent Application No.H6-69482
Patent Document 7 : Japanese Published Patent Application No.H7-265056

Non-patent Literature 1: Connect TissueRes.2003;44 Suppl 1:318-25
Non-patent Literature 2: Eur.J. Oral Sci. 1998; 106(Suppl.1):197-203

A transplantation of peripheral tissues to the defect portion of tissues by surgical resection is conventionally well performed. For the prosthesis of soft tissue, mechanical strength is not necessary. However for the bone defect of surgery accompanying bone resection, a prosthetic material, a structure as it is called, having mechanical strength is necessary. For the treatment of these defect portions, a filling material having an affinity to bone tissues such as *α*-phosphoric acid 3 calcium (*α* -TCP), *β*-phosphoric acid 3 calcium (*β*-TCP), hydroxyapatite were used.

From the view point of the regeneration treatment, not only the affinity to bone tissues, but also the attaching of osteoprogenitor cells to the prosthetic materials and the integration with peripheral bone tissues are expected. Therefore, the filling material is used after it is coated by the bone marrow fluid or after attaching the osteoblasts. Where the osteoblasts are provided by culturing the mesenchimal cells in vitro from the bone marrow fluid, seeding and culturing on to the filling material and differentiating into the osteblasts.

When such bone marrow fluid as described above is attached to the filling material, bone regeneration occurs by differentiation from marrow cells to bone cells with the expectation that effective mechanical strength can be obtained by integration with bone tissues. However, being dependent only upon the coating, the attached region of the mesenchimal cells stays only in the vicinity of the surface of the structure with the limited number of attachable mesenchimal cells, which may make the expected promoting effect of cure be unobtainable.

On the other hand, as detailed later, a method to impregnate a large amount of cells into the structure, after these cells are attached to the structure and an in-vitro cell culture is carried out, is studied in regeneration researches. In such a use of filling material, since a certain period is required for the cells to take root in a bone filling material which becomes a scaffold after the mesenchimal cells are seeded on the bone filling material. It is known that the mesenchimal cells have an inclination to congregate in the lower bottom of the filling material, and as a result, they become distributed unevenly or fallen away from the filling material causing failure to secure a sufficient number of mesenchimal cells or osetoblasts required for treatment.

To eliminate such a disadvantage, an artifice that the structure is made to be a porous structure in order to attach or to intertwine cells, or that a culture technique of the infiltration of cells from the structure surface is promoted. As a practical prior art, Patent Document 1 can be cited. In this prior art, a porous structure sintering a nonwoven fabric of a constant length and a constant cross-sectional dimension of titanium metal or titanium alloy, coated by cells is disclosed. According to this prior art, it is described that cells can be uniformly seeded on the surface, but there is no description that they infiltrated into the interior. It has been verified also in the additional tests by this inventor that in such a simple porous structure, cells stay on the surface and are hard to get in the interior deeply.

As an additional prior art, Patent Document 2 can be cited. In this art, a scaffold material composed of titanium or titanium alloy fabric sintered and fixed to the periphery of the implant of biological hard tissue is disclosed. This scaffold material enhances the infiltration of cells into the scaffold material and improves the affinity and fixity to the tissues.

This art has produced prominent results in dental materials and the like in which effects can be achieved even the thickness of the scaffold material is thin. However, when the scaffold material is thick, the advantage concerning the infiltration of cells is not reported.

In the infiltration of cells into the interior of the structure, generally speaking, when the size of the structure becomes large, the distance of infiltration of cells is limited and it was difficult for the cells to infiltrate the interior deeply, causing difficulties for the whole structure to change constructively into a new tissue of cell-built-in type. In order to solve such problems, this invention is directed first to provide a medical material having a structure which can diffuse and distribute a sufficient amount of substances inside of the material required for treatment.

### Disclosure of invention

The medical material of this invention comprises a structure formed from a plurality of jointed porous three dimensional structures with porosity of 20 to 97 %, the structure having a hole and/or a hollow section formed in a void between the porous three dimensional structures, wherein the hole and/or the hollow section is expanded dendritically inside the structure wherein the three dimensional porous structure is composed of one or more materials selected from a group of pure titanium, titanium alloy, cobalt chrome alloy, synthetic polymer, natural polymer, stainless steel, stainless alloy, aluminum and aluminum alloy, and wherein the material has a thin fiber shape part having thickness of 100 *µ*m or less. (claim 1).

The structure may be what has a porosity of which varies gradiently in a direction from a surface to an inside (claim 2). The structure may be what has at least one or more of detachable rigid bars penetrating the hole and/or the hollow section (claim 3).

In such medical material having the rigid bar, the thickness of the rigid bar is preferable to be 0.5 square centimeters or less in cross section area (claim 4), and the cross sectional shape of the rigid bar is preferable to have any of the shape selected from a round shape, an elliptical shape, a modified circular shape lacking a part of circular shape, polygon, variants of L-shape and H-shape etc. and hollow shape etc (claim 5).

In any of the medical material described above, the substance is preferable to be diffused and distributed inside of the structure by injecting the substance into the holes and/or hollow portions (claim 6). The substance diffused in the structure by injecting into the holes and/or hollow portions is preferable to be injected into the structure while being held by a gelatinous carrier. And the gelatinous carrier is preferable to be gelatine. Further, either calcium phosphate or ceramics apatite is preferable to be contained in at least a part of the structure (claim 7).

The structure may be made by laminating sheets having a part (claim 8). The structure may be made by laminating sheets having a thickness 0.1-to 10mm and may be hardened by pressurization or sintering (claim 10). And ceramics apatite is preferable to be coated on the periphery of the fibers composing the three dimensional structure (claim 9).

The substance injected into the structure has physiological function and is preferable to be at least one or more of those selected from the group composed of various cells, bone marrow cells, bone marrow fluids, stem cells separated from bone marrow fluid, umbilical cord blood derived cells, peripheral blood-derived cells, fragmented tissues, various proteins, lipids, polysaccharides, enzymes, antibiotics, antibacterial agents, hormones, cytokines, blood coagulation accelerants, cell growth promoting factors, extracts from genetically modified cells, substances produced from genetically modified cells, vascular endothelial cell growth factors (VEGF), platelet-induced growth factors (PIGF), transforming growth factors beta 1 (TGF. beta. 1), acid fibroblasts growth factor (aFGF), basic fibroblasts (bFGF), transforming growth factor (TGF. alph), epithelial cell growth factors, osteonectin, antiopoietin (ANG1), ANG2, blood platelet-derived growth factors AA, blood platelet-derived growth factors BB, bone morphogenetic proteins (BMP), hepatocyte growth factors (HGF), extracellular matrices, collagens, or complexes or derivatives of any of them (claim 11).

In the medical material of this invention, it is fundamental that the porous three dimensional structure having the porosity 20-97% has holes and/or hollow portions inside of the structure. Therefore, it is excellent in inductivity and in implantation performance of various cells commencing with anchorage dependent cells such as osteoblasts, and the growth potential is high.

The holes and/or hollow portions provided in the structure are distributed dendritically. Hence, it is easy to uniformly diffuse and distribute a sufficient amount of the growth factors of cell growth substance etc, required for treatment in the structure by interposing the holes and/or hollow portions. Such a void hole may be what is single or a plurality of independent holes.

Further, the holes or a part of the space may be opened or not opened to the external surface of the structure. To be specific, if a part of the hole is a straight hole and penetrates the structure, the later described rigid bar is easy to be inserted. If there is a hole or a space other than this through hole, it is possible to distribute the substance in the structure by injecting the object substance in the portion, and it is possible to fix the structure by utilizing the straight hole.

If the hole is opened, it is possible to directly inject the substance by applying the tip of a syringe to the portion. If it is not opened, it is possible to send the substance through the syringe by inserting the needle into the hollow or the hole.

Further, it is possible to seed cells even if the structure only has one opened hole. For example, one side of the hole is closed by a finger during the course of an operation, and it becomes possible to seed cells sufficiently inside of the structure by injecting cells etc. from the other side of the pore.

If the structure has such a distributed structure having hole or/and hollow section in the structure, it is possible to diffuse and distribute the substances uniformly in the structure by injecting a suspension or solution of a substance composing a tissue, tissue itself such as a thin tissue section, or a substance promoting tissue formation, into hole or/and hollow section. Hence, the medical material which can obtain efficient cell-built-in structure has been completed resultantly.

In order to make such porous three dimensional structure of the porosity 20-97% have holes and/or hollow portions inside of the structure, it is possible to obtain by perforating holes inside of the porous three dimensional structure, but since hollow portions can be secured by joining two or more of sheet-like or aggregated porous three dimensional structure, "porous three dimensional structure having holes and/or hollow portions" can be easily formed resultantly.

The above described three dimensional structure constitutes a structure whose shape is adapted to the portion in which it is used. Its porosity is 20-97%, and the shape of the structure and the selection of composing materials and porosity is needed to be set properly according to the required mechanical strength and the required cell distribution depending on the portion in which it is used. When the porous three dimensional structure is formed by at least more than two three dimensional bodies having different porosity, and when the three dimensional structure has a structure whose porosity inclinedly changes, it is possible to deal with the above described required performance extensively.

The porous three dimensional structure described above is made to be convenient in use, by preparing the structure whose size, shape, material, porosity etc. are adapted to the portion, if the portion in which it is used is determined previously. But, when there is no other choice than to determine the size adapted to the portion in which it is used during the course of an operation, it is possible to arrange the shape and the size of the structure adapted to the case by cutting out or sticking together the prepared structure with rough fitting of the size.

At this point, it is advantageous for the cells to promote the tissue growth to inject/charge sufficient amount of the required substance into inside of the porous three dimensional structure (the relevant structure), and the cells are needed to be distributed uniformly in the structure. In order to retain the injected substance as far as possible while uniformly distributing in the structure, it is preferable for the whole structure to be in a filtering state, to that end, the structure is preferable to have a porosity inclined from the surface to the inside of the structure, and it is particularly preferable for the porosity to have the porosity inclined being larger in the inside than in the outside. The structure having such a porosity inclination can be realized by preparing a plurality of structures of different porosity and by making a multilayer structure. Thereby, the porosity in the inside and the outside can be easily changed, and the inside of the structure is excellent in the filling factor of the injected substance in the structure, and the outside contacting a living body is capable of maintaining excellent inductivity and implantation performance of various cells, enabling the production of medical materials performing the each function.

The material of the structure is not particularly limited as far as the material has an affinity to cells. It is because the both cells of the bone tissue regeneration and the soft tissue prosthesis prefer the three dimensional structure. However, when the affinity to bone cells is expected, it is advantageous to contain either calcium phosphate or ceramics apatite in at least a part of the structure, because it enhances the affinity to bone cells.

Further, when the structure is an intertwined body of fibers, it is possible to maintain the strength high as well as to enhance the porosity. In such a case, the purpose is attained by using a fabric material composed of one or more of materials selected from the group of pure titanium, titanium alloy, cobalt chrome alloy, synthetic polymer, biological polymer, stainless, stainless alloy, aluminum, aluminum alloy etc. in at least a part of the structure. And it is preferable that ceramic apatite is coated on the periphery of the fabric material.

In order to manufacture such a structure specifically, a sheet of the thickness 0.1-10mm including at least the above fibers or composed of such fibers are laminated, pressurized, and sintered to fusion-bond between fibers, thereby it is possible to arrange the shape of the structure and to prevent from losing shape.

After producing the structure by this means, a substance to be injected into the structure is not particularly limited, but it is desirable to be cells and tissues having physiologically activity, proteins, and a substance suitable for exerting these functions. Specifically, the substance injected into the structure has physiology, and is preferable to be at least one or more of those selected from the group composed of various cells, bone marrow cells, bone marrow fluid, stem cells separated from bone marrow fluid, umbilical cord blood derived cells, peripheral blood-derived cells, thin tissue sections, various proteins, lipids, polysaccharides, enzymes, antibiotics, antibacterial agents, hormones, cytokines, blood coagulation accelerants, cell growth promoting factors, extracts form genetically modified cell, substances produced from genetically modified cells, vascular endothelial cell growth factors (VEGF), platelet-induced growth factors (PIGF), transforming growth factors beta 1 (TGF. beta. 1), acid fibroblasts growth factor (aFGF), basic fibroblasts growth factor (bFGF), transforming growth factors alpha (TGF. alph), epithelial cell growth factors, osteonectin, antiopoietin (ANG1), ANG2, blood platelet-derived growth factors AA, blood platelet-derived growth factors BB, bone morphogenetic proteins (BMP), hepatocytes growth factors (HGF), extracellular matrixes, collagens, or complexes or derivatives of any of them.

When the structure in which cells or physiological substances are embedded is needed to be fixed in the region of human body, it can easily be fixed by a suture used for general operative treatments. However, there may be a case where it is difficult to fix to the bone tissue only by suturing. In order to eliminate such a disadvantage, the structure having one or more hole penetrating the structure is presented in this invention. This hole is not limited to one, and it is possible to provide even two or three when needed.

So when the structure comprises penetrating hole, the structure may be fixed to the bone tissues by stabbing the rigid bar inserted in the penetrating hole into adjoining bone tissues.

In this occasion, it is important that the relation between the structure and the rigid bar is detachable and not integrated. As a similar prior art, there is Patent Document 1. In this, a nonwoven fabric made of titanium metal or titanium alloy fabric is wound around outside of a stem made of titanium and integrated by sintering. Accordingly, the both sides are not detachable in this prior art.

In the medical material of this invention, it is possible to prepare filling materials of the size corresponding to the case by making the structure be pierced in the rigid bar, when the space is required to be filled with the structure. The relation between the rigid bar and the structure being never integrated and being detachable allows the filling and the fixing of the structure into the space. While the material disclosed in the Patent Document 1 cannot be used.

In the prior art of Patent Document 1, there is no description of the size and the shape of the rigid bar or the stem made of titanium, and a drawing to use for the enthesis of a defect in a femur is described as an example. However, these stem is not suitable for babies and may only be usable for adults. It is because if bone enthesis is carried out to the babies in the period of growth, bone formation adapting to the growth can not be promoted. Therefore, in this case, the thickness of approximately 1 centimeter or larger is necessary for strength, though there is no description of the diameter of the cross section of the stem in the prior arts.

In the medical material of this invention, since the rigid bar and the stem are extraneous material for a living body, an intent study has been done to thin the thickness of the rigid bar as far as possible and for integrating with the bone tissues. The reducing of the total amount of the extraneous material lowers the extraneous property. Resultantly, the necessity for thinning the thickness of the rigid bar as far as possible and for reducing the amount of foreign body was made to be apparent. Further considering the problem in view of the strength it is not preferable if it is too much thinned. The preferable size of the cross sectional area is limited to be 0.5 square centimeter or less.

However, since nothing but to thin it will result in insufficient strength, this invention employed to incorporate artifices of mechanical engineering such as to make the cross section be L-shaped, H-shaped, etc. Further, there is a possibility for the structure to rotate about the rigid bar if it is made in a circular cross section. It is preferable to make the rigid bar having a non-circular shape (for example, ellipse, polygon etc.) to prevent unnecessary motion.

Depending on the portion where it is used, there is also a case that such rotation does not cause a problem. In such a case, the cross section of the rigid bar may be a circular shape, a modified circular shape lacking a part of circular shape. Therefore, in the medical material of this invention, the circular cross section is not excluded.

Depending on the portion where it is used, the enthesis adapted to the size of defect portion by jointing fish sausage-like structures is possible. However it can be encountered in the scene of operative treatments, that the more complicated size adaptation is required.

For example, in the region of orthopedic surgery in autotransplantation of bones, bone chips are harvested from an iliac bone. A defect arises in the portion of the iliac bone which can be filled in by a plastic material. However the stability in the affinity is raised as a problem, when the plastic materials are used.

In such a case, at least one or more of the rigid bar are used and laid over the defect portion of the iliac bone as if they are bridged. After that, two sheet-like porous three dimensional structures are cut tailoring to the size of the defect portion, and they are stuck together by interleaving the rigid bar. Or one of a large porous three dimensional structure sheet may be folded to interleave the rigid bar and the sheets can be knitted up mutually with a suture or a wire before fixed, thereby enabling to prepare a porous three dimensional structure having the most suitable size and shape for the case.

Carrying out such a work makes a hollow portion in the void between the two porous three dimensional structures. A hollow will be formed at least in the vicinity of the rigid bar. This condition coincides with the condition cited in this invention that " the porous three dimensional structures has holes and/or hollow portions provided inside of the structure."

In this condition, it becomes possible to inject cell and cell growth factors into the holes and/or the hollow portions and to distribute uniformly from the inside of the three dimensional structure sheet.

In such process of cell seeding or infiltration of substances by sticking together of the sheets, there is also a merit to enable more easy and pervasive treatment. Because the treatment may be done on the one side prior to the sticking together and the treated faces may mutually stuck afterwards.

In the medical material of this invention, it is made to be possible to distribute various cells and cell growth factors inside of the structure not only while in an operative treatment as described above, but also before the operative treatment, further it is possible to do it extracorporeally such as cell culture etc.

For example, when hydroxyapatite is made to be distributed uniformly inside of the structure before an operative treatment, it is possible to precipitate them superposedly inside of the structure, by injecting the solutions containing those into the holes or hollow portions repeatedly.

Further on one hand, by injecting the suspension of cells or fragmation into the holes and/or hollows repeatedly, they can be precipitated superposedly inside of the structure. And it is possible to drastically form a cell assembly in the three dimensional structure by cell culturing.

The three dimensional cell assembly thus produced can be utilized as an advantageous structure to make cells which produce various substances depending on the kinds of cells proliferated there. Accordingly, it becomes advantageous porous three dimensional structure to make exert the special function of various genetically manipulated cells.

Such artifices to maintain porous and three dimensional structure has bee carried out up to now so as to be advantageous for the circulation of culture solutions to cells as well as to utilize the cell functions by culturing a large amount of cells three dimensionally. For example, an artifice is reported as a prior art that a porous structure is produced three dimensionally using collagen etc. and make them have an affinity to cells.

They can culture a large amount of cells and achieves the purpose, but there is a problem that the three dimensional structure deforms due to a long period of culture. And as the result, the intercellular space becomes narrow, and the reflux solution for cell culture becomes hard to flow evenly between the cells and happens to cause troubles not only in the proliferation of cells but also in exerting the function.

As for its cause, it is understood that the culture cells secrete fibers comfortable for collagen etc. around the cells, and the gradual contraction of those fibers or the hauling of these fibers by the cells forces the whole tissue to contractile.

Therefore, when such a three dimensional structure is formed by collagen, there happens a case that cannot attain the purpose due to the deformation of above contraction.

The medical material of this invention has a merit that it does not deform easily because it is made of titanium or titanium alloy. Thereby a disadvantageous condition due to the contraction won't rise up. Further it is even more undeformable when it is sintered.

At the same time, medical material of this invention has the three dimensional structure having firm shape retention due to the use of titanium or titanium alloy and further sintered. Therefore, it is possible to prepare a matrix for cells securing the shape retention property while enhancing an affinity to cells by impregnating or confounding synthetic polymer material such as micro polyester fibers or natural polymer material such as collagen fibers. Moreover, the coating with apatite may be carried out on the periphery of the fibers composing the three dimensional structure. Thereby, the affinity to cells is furthermore enhanced, realizing early induction of cells.

Thus, the three dimensional porous structure containing a large amount of cells can be embedded directly in a living body if the kind and the purpose of the operative treatment coincide.

And, by carrying out a long period of cell culture in the original condition, it can be functioned as a reactor utilizing cells.

Further, in the medical material of this invention, cells, thin sections of cell, and/or physiological substances etc. may be contained in a gelatinous carrier, and injected into holes and/or hollow portions of the structure. Thereby, the injected cells and physiological substance etc. are retained in the structure and gradually diffused inside of the whole structure. Hence, these substances can be uniformly and efficiently diffused in the structure.

As such gelatinous carrier, followings can be cited: in biodegradable polymers; for example, polylactic acid composed of natural or synthetic polypeptide, poly glycolic acid, polyorthoester, polyacid antihydrate, or gelatine, chitin, collagen, agarose, microbial polysaccharide (pullulan, curdlan): in chemical synthesis system; polypeptide, synthetic polysaccharide, aliphatic polyester (polycaprolactone, polybutylene succinate, poly glycoric acid, polylactic acid), polyvinyl alcohol, polyaminoacetic acid(PMLG): in natural system; chitosan as animality origin, starch, cellulose, acetycellulose etc. of plant origin. They can be used by mixing one or more than two kinds. Particularly, gelatine or collagen is preferable because they have high bio-compatibility.
Moreover, gelatine and collagen can produce hydrogel by bridging, and inside of the hydrogel, water solution of cell growth factors can be easily fixed. Further, the hydrogel is decomposed in a living body with time, and since its decomposition rate can be controlled by the degree of bridging of the hydrogel, the cell growth factors fixed in the hydrogel can be efficiently time-released.

As described above, this invention not only solved the problems of conventional medical material but also enabled to expand the application of medical materials to new fields.

On the one hand, the natural tooth is composed of a tooth and a periodontal tissue, and the periodontal tissue is composed of cementum, gingiva, alveolar bone, periodontal membrane. Among them, the periodontal membrane is composed of collagen fibers running vertically to the tooth root and the alveolar bone, and it tightly joints the tooth root and the alveolar bone, playing an important role in the lives of human such as a buffer for occlusal force, perception, adjustment of nerve, mastication motion etc. However, in a conventional artificial tooth root, because it is fixed directly to a jaw bone without a periodontal membrane, the shock in mastication is directly transmitted to the jaw bone, concentrating excess stress to the alveolar bone. As the result, there is a problem that there occurs the coming off or loose of the implant by bone resorption.

In order to solve such problems, an artificial tooth root equipped with various artificial periodontal membrane is under review. In Patent Document 3, an artificial tooth root is disclosed, in which a support medium composed of an organic material supporting material provided by culturing the extracted tooth harvested from human periodontal cells, is attached as the artificial periodontal membrane.

In Patent Document 4, an artificial tooth root is disclosed, in which a protein component for artificial periodontal membrane, containing protein including hydroxyproline residue is attached and supported to an organic absorption membrane.

Document 5, an artificial tooth root is disclosed, in which an organic resin membrane having numbers of pore on top of the micro projection formed in at least one side of the intraosteal implant anchor of an artificial tooth root, is covered.

However, the implanting method utilizing cultured periodontal membrane cells as disclosed in Paten Document 3 leaves a problem that the periodontal membrane is hard to obtain and further a complicated operation to culture the obtained periodontal membrane is required.

Moreover, the artificial tooth root of Patent Document 4, supporting a material for forming periodontal membrane, or the artificial tooth root of Patent Document 5, in which the intraosteal implant anchor having micro projection is covered by the organic resin membrane having numbers of pore, can regenerate an artificial periodontal membrane. However, these artificial tooth root leave a problem that they do not have enough strength to withstand the mastication force or mastication motion of human, in which the binding strength between the artificial tooth root and the bone tissues is said to be an average 60 kg.

The inventor describes in Patent Document 2 that "The osteoblasts growth preferably in geometric spaces composed of thin fibers, and shows an extremely high affinity and has a property to adhere positively to the structure which is a geometric space composed by titanium fiber group of the thickness 100 *µ*m or less with its expanse being 100-400 *µ*m." Based on this knowledge, the inventor discloses an biological pore tissue inducing scaffold material composed of a rod and a biological tissue implanting space which is made by winding the layer formed by intertwining and layering titanium fibers or titanium alloy fibers having a diameter of 100 *µ*m or less and aspect ratio of 20 or more (short axis: long axis=1:20 or more), around the periphery of the rod. By the using the scaffold material of Patent Document 2 in a living body, a three dimensional physical binding is formed between the material and the osteoblasts, to become extremely good osteo-integration tissue. The layer in which bones and metal is integrated and coexistent is called to be a collaboration zone.

The implant material of Patent Document 4 is equipped with a porous biological tissue implanting layer, so the collaboration zone with the osteoblast will form and bind firmly with a living body. However, the periodontal membrane of this implant does not have the buffering function because it does not have the collagen fibers running vertically to the tooth root and the alveolar bone like the natural tooth root.
Meanwhile, a honeycomb structure membrane having thousands of a straight tunnel of the diameter 200 *µ*m is known for growing the collagen fiber perpendicular on the membrane surface, well as growing the blood vessels and nerves perpendicular (Non-patent Literature 1).

### Brief Description of Drawings

[Figure 1] Figure 1a, 1b, 1c are perspective views showing an embodiment of the medical material of this invention.
[Figure 2] Figure 2 is a perspective view showing the other embodiment of the medical material of this invention.
[Figure 3] Figure 3 is a perspective view showing further the other embodiment of the medical material of this invention.
[Figure 4] Figure 4 is a perspective view showing further the other embodiment of the medical material of this invention.
[Figure 5] Figure 5 is a perspective view showing further the other embodiment of the medical material of this invention.
[Figure 6] Figure 6a, 6b, 6c are perspective views showing further the other embodiment of the medical material of this invention.
[Figure 7] Figure 7a to Figure 7d are perspective views showing an embodiment of the rigid bar usable in the medical material of this invention.
[Figure 8] Figure 8a is a perspective view showing a healthy iliac bone portion, and Figure 8b is a perspective view showing a iliac bone in which a defect portion is formed.
[Figure 9] Figure 9a is a perspective view of a status in which the rigid bar is fixed to the defect portion of the iliac bone portion of Figure 8b, Figure 9b is a perspective view just prior to folding the sheet-like three dimensional structure to the defect portion of Figure 9a, and Figure 9c is a perspective view after folding the three dimensional structure to the defect portion of Figure 9a.
[Figure 10] Figure 10a is a side cross sectional view of a status in which the rigid bar is fixed to the defect portion of the iliac bone portion of Figure 9a, Figure 10b is a side cross sectional view just prior to folding the sheet-like three dimensional structure to the defect portion of Figure 9a, and Figure 10c is a side cross sectional view after folding the three dimensional structure to the defect portion of Figure 9a.
[Figure 11] Figure 11a, 11b are outline drawings showing when the medical material of this invention is used in the repair of a cranial bone.
[Figure 12] Figure 12a, 12b are outline drawings showing when the medical material of this invention is used in the repair of a tooth in a carious tooth portion.
[Figure 13] Figure 13 is a cross sectional view showing an embodiment of the artificial tooth root of this invention.
[Figure 14] Figure 14 is a cross sectional view showing further the other embodiment of the artificial tooth root of this invention.
[Figure 15] Figure 15a is a perspective view showing an embodiment of the cell culture platform of this invention, Figure 15b is an enlarged drawing thereof, and Figure 15c is a perspective view showing the holder into which the cell culture platform of this invention is inserted.

### Best mode for carrying out the invention

Next, the medical material of this invention is described using drawings.

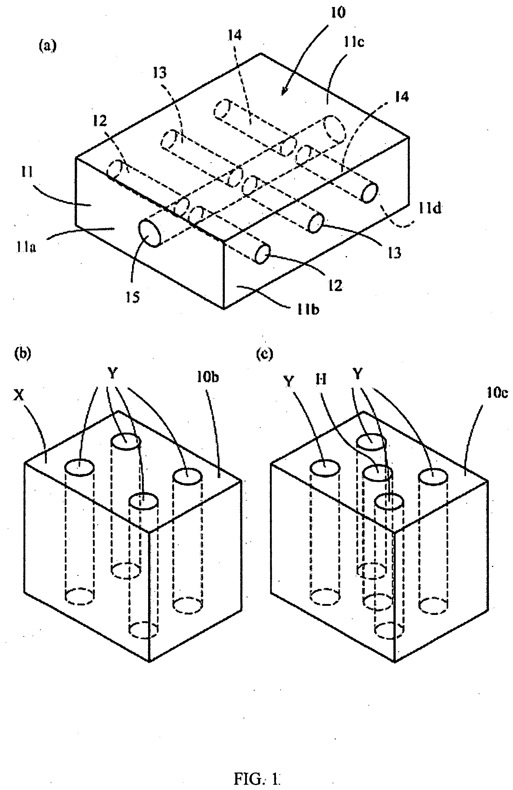

The medical material 10 shown in Figure 1a comprises a porous three dimensional structure 11 of rectangular parallelepiped shape whose porosity is 20-97%, and holes 12, 13, 14, 15 formed inside of the body.

The structure 11 is equipped with two facing small sides 11a, two facing large sides 11b, a top face 11c, and a bottom 11d.

The structure 11 is composed of pure titanium, titanium alloy, cobalt chrome alloy, synthetic polymer, natural polymer, stainless, stainless alloy, aluminum, aluminum alloy etc., and it may be composed of a plurality of them. The three dimensional structure whose porosity is 20-97% is formed by intertwining thin fiber-like material of the thickness 100 *µ*m or less. The thickness of the structure is formed into sheet-like shape of the thickness 0.1-10mm. It is preferable to solidify by pressurization or sintering when it is formed by the above metals. Moreover, it may be coated by apatite composed of hydroxyapatite containing carbonate apatite and other calcium phosphate in the periphery of the fibers. Thereby, the affinity to cells is further enhanced enabling to obtain medical materials which is high in cell induction property and binding capacity with cells.

The hole 15 is a cylindrical one formed in parallel to the large side 11, penetrating the center of the both small sides 11a. And, the holes 12, 13, 14 are formed in parallel to the small side 11a, penetrating the both large sides 11b, and it is a cylindrical one communicating with the hole 15 in the center portion. These holes 12, 13, 14, 15 all penetrate the structure 11. In the medical material 10, the holes are distributed dendritically around the hole 15 as the axis. The holes 12, 13, 14, 15 may be all regarded to be one hole.

The medical material 10 can introduce or inject a promoting substance of cell growth having excellent in bio-compatibility like calcium phosphate, ceramic apatite and etc, because it has a hole in the inside that communicate with the outside. So, the introduced substance can be diffused in the structure which is different from those known in the prior art. As the substance to be introduced or injected, physiologically active cells and tissues, proteins, and substances that are appropriate to make exert those functions can be used. Specifically, the substance injected into the structure has physiology, and various cells, bone marrow cells, bone marrow fluid, stem cells segregated from bone marrow fluid, umbilical cord blood derived cells, peripheral blood-derived cells, thin tissue sections, various proteins, lipids, polysaccharides, enzymes, antibiotics, antibacterial agents, hormones, cytokines, blood coagulation accelerants, cell growth promoting factors, extracts form genetically modified cell, substances produced from genetically modified cells, vascular endothelial cell growth factors (VEGF), platelet-induced growth factors (PIGF), therapeutic gain factors beta 1 (TGF. beta. 1), acid fibroblasts (aFGF), basic fibroblasts (bFGF), therapeutic gain factors alpha (TGF. alph), epithelial cell growth factors, osteonectin, antiopoietin (ANG1), ANG2, blood platelet-derived growth factors AA, blood platelet-derived growth factors BB, bone morphogenetic proteins, hepatocytes cell growth factors (HGF), extracellular matrices, collagens, can be cited.

The substance introduced in the structure by injecting into hole may be contained or held in a gelatinous carrier. Thereby, the liquid introduced substance will not flow out of the structure. And, since the substances are gradually diffused uniformly with sustained-release inside of the whole structure, the introduced substances can be efficiently worked.

As such a gelatinous carrier, the above described biodegradable polymers such as gelatin, collagen can be cited. Hydrogel produced by bridging gelatine and collagen can fix water solution of cell growth factors in the polymer hydrogel. Further, the hydrogel will decompose with time, and its rate of decomposition can be controlled by the degree of bridging of the hydrogel, which is preferable.

To the structure in which hydroxyapatite coating is carried out in the periphery of the fiber of the three dimensional structure composed of fiber shape, since it is possible to increase the amount of integration of gelatine microsphere, the injection amount of the introduced substance can be increased.

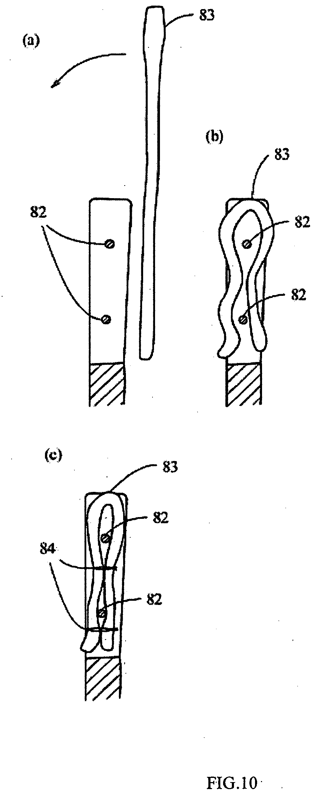

Further, the medical material 10b shown in Figure 1b is composed of the porous three dimensional structure made from three dimensional body X, Y of different porosity. When the porosity of the three dimensional body X of the medical material 10b is larger than that of the three dimensional body Y, the three dimensional body Y works as a supporting column of the structure and it increases the mechanical strength of the structure. And, when the porosity of the three dimensional body X is smaller than that of the three dimensional body Y, the cells and cell growth factor can be induced in body Y which enables the tissue to promptly formed in the whole structure. It is because the regeneration of cell occurs in the inside and the cell induction occurs from the outside. Further, the three dimensional body of different porosity composing the structure shown in Figure 1b may be formed so that the porosity changes at a slant from the three dimensional body X to the three dimensional body Y. In this case, since the porosity changes smoothly, cells are easily grown in the direction toward the low porosity. Further, the medical material shown in Figure 10c may have a hole H formed in the porous three dimensional structure formed from the three dimensional body X, Y of different porosity.

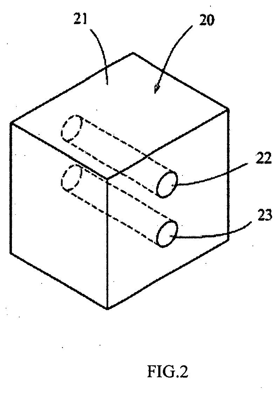

The medical material 20 shown in Figure 2 comprises a porous three dimensional structure 21 and holes 22, 23 formed inside of the body. Here, the hole 22 and the hole 23 are independent mutually. The other composition is the same with the medical material 10 of the figure 1. In this embodiment, the holes are formed independently so as to be in parallel mutually, but it may be in different directions. To have such independent holes enables to diffuse the introduced substance widely in the structure.

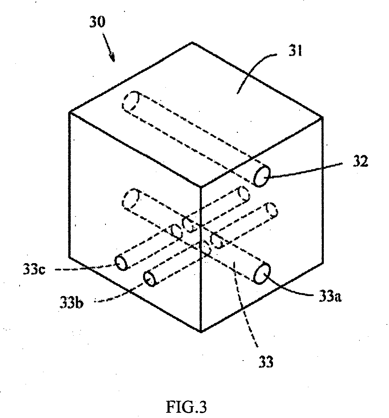

The medical material 30 shown in Figure 3 comprises a porous three dimensional structure 31 and holes 32, 33 formed inside of the body. The hole 32 and the hole 33 of this medical material are also independent mutually. The hole 33 is distributed dendritically similar to the holes of Figure 1a, and composed of small hole 33a and small holes 33b, 33c provided perpendicular to the small hole 33a joining in the center of the small hole 33a. The other composition is the same with the medical material 10 shown in Figure 1a.

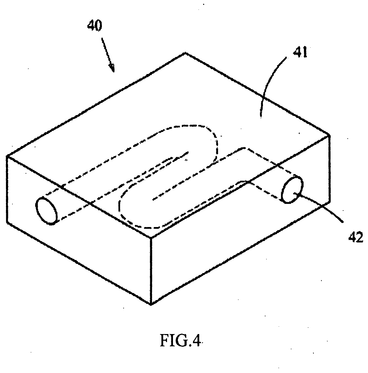

The medical material 40 shown in Figure 4 is provided with a hole 42 penetrating the three dimensional structure 41. In the previous embodiments, the holes are all linear shape, but this hole 42 has inverse S-shaped curve. Thus, the holes of the medical material of this invention may have any shape, thereby can diffuse the introduced substance in the structure.

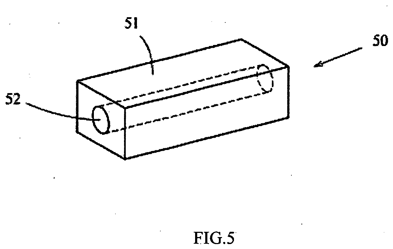

The medical material 50 shown in Figure 5 is provided with a cylindrical hole 52 formed in the center of the rectangular parallelepiped-shape porous three dimensional structure 51.

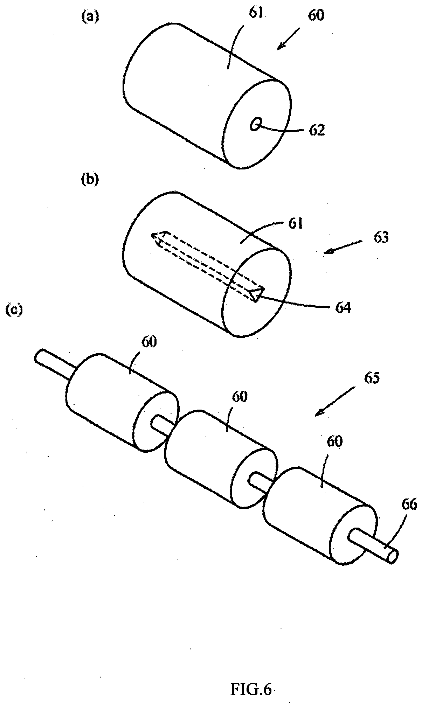

The medical material 60 shown in Figure 6a is provided with a cylindrical hole 62 formed in the center of the column-shaped porous three dimensional structure 61. And, the medical material shown in Figure 6b is provided with a hole 64 whose cross section is triangle formed in the center of the porous three dimensional structure 61.

The medical material 65 shown in Figure 6c comprises the medical material 60 or 63 and rigid bar 66 interlinked with medical material 60 or 63. In this medical material 65, the rigid bar 66 is equipped detachably so as to be adjustable into the size of the medical material fitted for the case in the site of operation etc. Moreover, in the case of operation of a costa and a fibula, the medical material 65 can be easily fixed to the body using rigid bar 66.

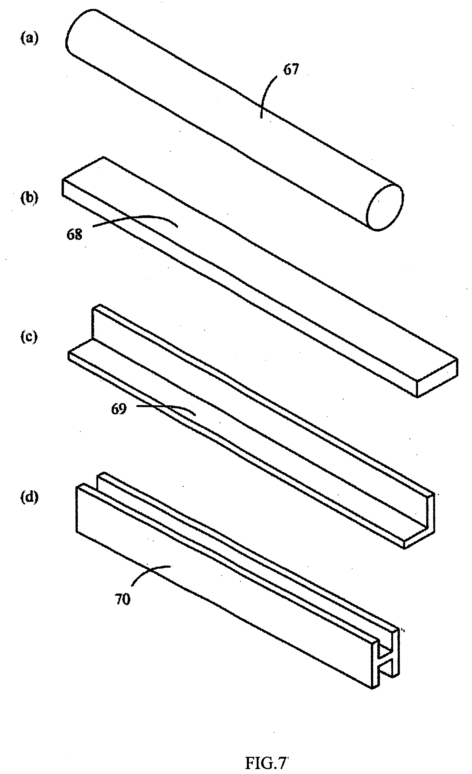

In Figure 7a-Figure 7c, the other embodiments of the rigid bar usable in the medical material 65 are shown. The rigid bar 67 shown in Figure 7a is circular in cross section, the rigid bar 68 shown in Figure 7b is rectangular in cross section, the rigid bar 69 shown in Figure 7c is L-shaped in cross section, and the rigid bar 70 shown in Figure 7d is H-shaped in cross section. These rigid bars can be selected arbitrary according to the position where the medical material is placed. When a medical material having high strength is needed, the rigid bars 69, 70 are preferable. Since the rigid bar is detachable, the medical material 65 may be formed by inducing the substance into the medical material 60 or 63 and then inserting rigid bar into the medical material 60 or 63. By making the shape of the rigid material be what is different from the shape of the hole of the medical material or be what has a clearance in between, it is possible also to insert injection syringe etc. into the clearance formed by the difference of the shapes after the rigid bar is inserted into the medical material and feed in the introduced substance.

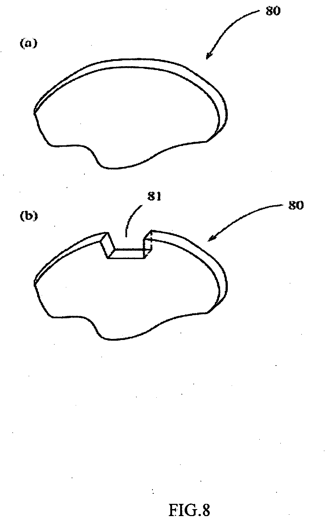

Figure 8-Figure 10 shows the method to use the medical material of this invention in the orthopedic surgery fields, in the case when a defect portion in an iliac bone is generated by autogenous bone graft in orthopedic surgery fields.

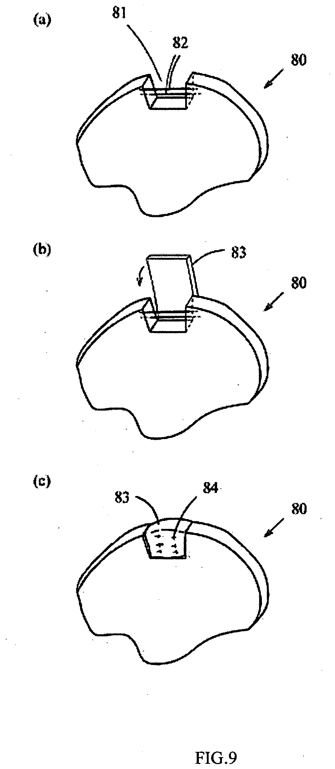

The reference numeral 80 of Figure 8a is a healthy iliac bone portion. By performing autogenous graft of this healthy iliac bone, the defect portion 81 is formed as shown in Figure 8b. Here, the rigid bar 82 is fixed in the defect so as to bridge it (see Figure 9a). In this case, the two rigid bars 82 are used but the number is selected according to the largeness of the defect portion.

Next, the sheet-like formed porous three dimensional structure 83 of the porosity 20-97% is cut out in conformity to the size of the defect portion (see Figure 10b). And then the porous three dimensional structure 83 is folded so as to cover the defect portion 81 and to tuck the rigid bar 82 (see Figure 10b). The folded porous three dimensional structure 83 is sewn together by a line 84 such as suture or wire to fix (see Figure 9c, Figure 10c).

Thus, a hollow is formed in the clearance of the folded sheet-like porous three dimensional structure 83. And at least in the vicinity of the rigid bar, a hole is formed.

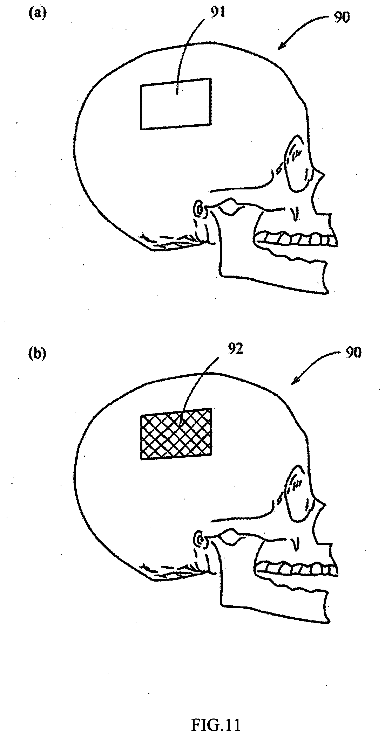

In Figure 11a, b, the medical material of this invention is used for repair of a cranial bone.

The reference numeral 90 of Figure 11a shows the cranial bone, the reference numeral 91 shows its defect portion. Figure 11b shows that one sheet of the medical material 92 having holes is inserted into the defect portion 91. Here, the medical material 92 is substantially the same with the medical material 10 of Figure 1a. However, the shape of holes formed in the medical material 92 is not particularly limited. It can be determined arbitrarily in conformity to the largeness of the defect portion. By inserting a sufficient amount of the medical material of growth factor such as cell growth substance etc necessary for the treatment into the holes and by inserting the medical material 92 into the cranial bone, early repair of the defect portion can be expected. It is because, the growth factors will be diffused and distributed uniformly in the structure.

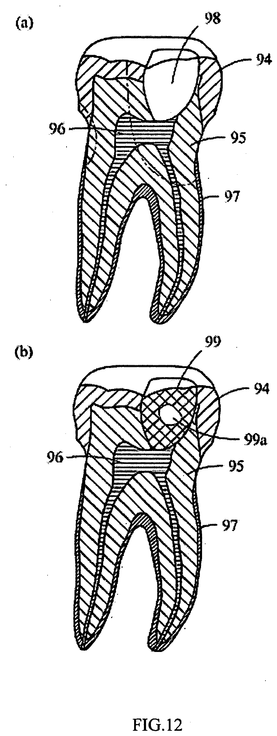

Figure 12a, b shows a method for repairing the portion of a carious tooth by filling the medical material of this invention.

The reference numeral 94 of Figure 12a shows an enamelum portion of a tooth, the reference numeral 95 shows a dentinum portion of the tooth, the reference numeral 96 shows a dentinal pulp, and the reference numeral 97 shows a cementum of the tooth. The reference numeral 98 shows a portion of the carious tooth. Conventionally, the treatment of serious carious tooth advanced to the dental pulp is as follows; grinding down the portion of the carious tooth; filling in calcium hydroxide in the carious tooth portion, killing bacteria of the carious tooth, and preventing the leak of dental pulp by the dead bacteria. Further, a crown is provided so as to cover the formed calcium hydroxide filled in the carious tooth.

In Figure 12b, a medical material 99 having a hole 99a is formed into the shape of the portion of a carious tooth and filled in a carious portion 98. The other composition of the medical material 99 is substantially the same with the medical material of Figure 1a. By using this dimensional structure 99 which the bone regeneration factors or cells are injected into the hole 99a, the formation of the tooth from inside of the medical material 99 and formation of the tooth from outside by the dental pulp 96 are carried out. Thereby the tooth (dentinum, enamelum, (further, in the case of serious carious tooth as shown by imaginary line in Figure 12a, or in the case of carious tooth in the side of tooth, cementum)) is early formed to repair the portion 98 of the carious tooth. Moreover, other than the growth factor of a sufficient amount of cell growth substance necessary for treatment, a germicidal agent of bacteria of carious tooth used for treatments of carious tooth may be injected in the hole 99a of the medical material, and filled the carious tooth in the portion 98 afterward.

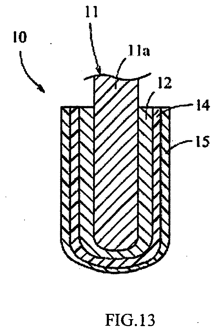

Next, the artificial tooth root of this invention is described.
The artificial tooth root 100 of this invention shown in Figure 13 comprises a substrate 101, a metal fiber layer 102 provided in the periphery of the substrate, a metal wire fiber layer 103 provided in the periphery of the metal fiber layer, a periodontal membrane forming layer 104 provided in the periphery of the metal wire fiber layer, and an alveolar bone forming layer 105 provided in the periphery of the absorbable polymer film.

The substrate 101 is a column-shaped metal rod whose diameter is 1-5mm, and length is 5-20mm. This metal substrate is made of metal having high bio-compatibility, and particularly, titanium, titanium alloy, gold, gold alloy can be cited. The upper portion 101a of the substrate protrudes from the gum-ridge and engages with the artificial tooth root (not shown in the figure).

The metal fiber layer 102 is formed by winding a nonwoven fiber made of metal wires around the periphery of the metal substrate 101. This nonwoven fiber is made by interwinding the metal wires having diameter 5-100 *µ*m to make the porosity be 50-90%. Such nonwoven metal fiber has a three dimensional structure in which osteoblasts preferably take root, and the metal fiber layer 102 composed of nonwoven fabrics works as a hard tissue inducing layer which induces osteoblasts.

In other words, the osteoblasts induced to the metal fiber layer (metal nonwoven fabrics) are differentiated and induced in the metal fiber layer and forms a hard tissue layer. The artificial tooth root and the living body are firmly fixed, because the osteoblast forming hard tissue layer and the metal fiber wire (metal nonwoven fabrics) are physically associated.

This metal fiber layer 102 is made of a metal having high bio-compatibility same with the metal substrate, especially titanium, titanium alloy, gold, gold alloy. Thereby it enhances the induction and settlement of osteoblasts. Further, the welded binding of the substrate and the metal fiber layer by vacuum sintering as later described may be strengthen, if the material of the metal fiber layer and the substrate are same material.

The periodontal membrane forming layer 104 is formed of biodegradable polymer and presents a honeycomb structure in which a large number of holes of the diameter 100-400 *µ*m is formed perpendicular to the axis of the metal substrate. In other words, undifferentiated mesenchymal cells induced into periodontal membrane forming layer 104 is differentiated and induced into fibroblasts of collagen fibers (Charpy fiber)etc. in the periodontal membrane forming layer. And, since this periodontal membrane forming layer has the honeycomb structure having holes formed in the direction perpendicular to the axis of the substrate, these collagen fibers grow in the direction perpendicular to the hard tissues and the alveolar bone. As a result it presents the same shape and structure with the periodontal membrane existing in a natural tooth.

As the biodegradable material used for the periodontal membrane forming layer 104, porous membrane, collagen fiber membrane made of biodegradable plastic such as polylactic acid, polycaprolactone copolymer, and a complex of those and cytokine such as bone morphogenetic protein and fibroblast growth factor can be cited. Preferably, polylactic acid/polycaprolactone copolymer or a complex of porous membrane and cytokine are used.

In order to produce these membrane, the solvent of the material is formed into membranous shape on a flat plate of glass, plastic or metal with the thickness 50-1000 *µ*m or preferably 100-300 *µ*m. For example, when polymer is used as the material, the material is dissolved in an organic solvent and evaporated on the flat plane. Or, when it is collagen, moisture in the gel is freeze-dried on the flat plate to form a film. The details of the producing method using biodegradable plastic polymer is described in Non-Patent Document 1, and the details of the producing method for periodontal membrane using collagen is described in Non-Patent Document 2, and any of which is publicly known method. After forming membrane layer, through holes whose diameter is 100-400 *µ*m and the rate of the hole are is 40-70%, preferably 50-60%, are formed using the physical means, such as a laser perforation cited in non-patent Document 1 or the perforation by a needle. The cross section of through hole is usually circular, but it may be triangular, rectangular or hexagon. The similar membranes are partly available commercially.

The alveolar bone forming layer 105 is formed of biodegradable porous polymer membrane and do not have the honeycomb structure. By providing such periodontal membrane forming layer 105, repair of the alveolar bone having large defect can be promoted. This alveolar bone forming layer 105 is formed by winding the porous polymer membrane around the periphery of the periodontal membrane forming layer 104 and by adhering with adhesive agents etc.

The artificial tooth root 100 is produced as follows; at the beginning, a substrate of metal rod 101 is prepared; nonwoven fabrics which is made by intertwining and sintering metal wires used for a metal fiber layer 102 are wound around the periphery of the titanium rod; further metal wires used for metal wire fiber layer 103 are wound; it is loaded in a sintering jig tube made of ceramic and is sintered about 0.5-5 hours at the temperature about 500-1500 °C in vacuo with the degree of vacuum about 10⁻³ Pa or more; the complex produced as such is welded in its mutual contact point of the metal, and its contact point of the metal rod and the metal wire (nonwoven fabric) becomes strong; then, the membrane used for the periodontal membrane forming layer 104 is wound around the periphery of complex; further, the nonwoven fabrics used for the alveolar bone forming layer is wound to produce the artificial tooth root 100.

Any or all of the metal fiber layer 102, metal wire fiber layer 103, periodontal membrane forming layer 104, alveolar bone membrane forming layer 105 may support cytokine, by immersing any or all the layer in a solution containing cytokine such as bone morphogenetic protein (BMP) and fibroblast growth factor (FGF). Further, the hydroxyapatite may be supported in the outer circumference of the absorptive polymer membrane by using a solution containing hydroxypatite. These supporting method are not particularly limited.

By implanting the artificial tooth root in a living body, collagen fibers are formed in the periodontal membrane forming layer, and a hard tissue layers are formed in the metal fiber layer. Thereby, the artificial tooth root is firmly bound to the living body, and artificial periodontal membrane is formd between the artificial tooth root and the living body, preventing the dropping out or loose of the artificial tooth root. The artificial tooth root regenerated by this periodontal membrane is substantially the same with the natural periodontal membrane except that the dentinum of the periodontal membrane is replace by the metal.

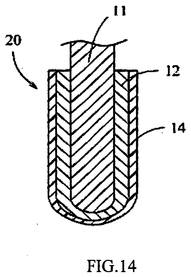

In the artificial tooth root 110 shown in Figure 14, the alveolar bone forming layer 105 is abbreviated. The other composition of the artificial tooth root 110 is substantially the same with the artificial tooth root 100 of Figure 13.

The artificial tooth root 110 is produced as follows; nonwoven fabrics used for a metal fiber layer are wound around the periphery of the substrate 101; it is loaded in a sintering jig tube made of ceramic and is sintered about 0.5-5 hours at the temperature about 500-1500 °C in high vacuo with the degree of vacuum about 10⁻³ Pa or more; the artificial tooth root 110 is produced by winding the membrane used for the periodontal membrane forming layer 104 around the complex thus produced.

Even when the alveolar bone forming layer 105 is not equipped as just described, collagen fibers are formed in the periodontal membrane forming layer by implanting the artificial tooth root 110 in a living body, and hard tissue layers are formed in the metal fiber layer, the artificial tooth root and the living body bind firmly.

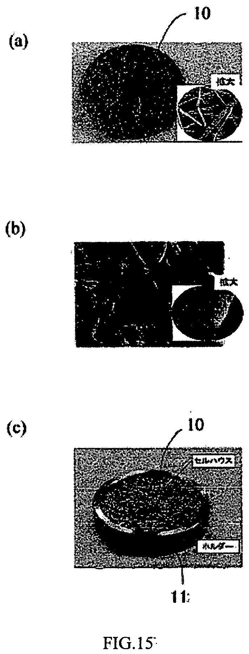

Next, the producing method of the medical material of this invention is described. A cell house (cell culture platform) 120 of Figure 15 is a disc whose diameter is 0.3-3mm, and is produced as follows. A titanium three dimensional structure made by intertwining titanium wire of the thickness 0.3-3mm, the diameter 100 *µ*m or less so as to make the porosity be 50-95% is heated in atmosphere at a temperature 400-800 degree, preferably 550-600 degree until it presents purple, and after the heating, it is immersed in hydroxyapatite formation liquid for 48 hours to adhere hydroxyapatite.

In this titanium three dimensional structure, titanium metal wire of the diameter 100 *µ*m or less is compressed into a previously prepared circular mold so as to make the porosity be 50-95% and is further vacuum-sintered.

The titanium three dimensional structure thus formed is heated in atmosphere at a temperature 550-600 degree. This heating is carried out until the titanium three dimensional structure presents purple. Thereby, the surface of the three dimensional structure is covered with an oxide film of the thickness 600-800 Å. Such titanium three dimensional structure covered by the oxide film adheres to hydroxyapatite firmly and has sustained release property, which is preferable.

As such hydroxyapatite, a sintered compact of hydroxyapatite, hydroxyapatite containing carbonate apatite can be cited. The hydroxyapatite formation liquid contains phosphoric acid ion and calcium ion.

Since the cell culture platform produced by the producing method of this invention is configured as described above, by the three dimensional effect, it can differentiate, induce various cells, promotes production of useful substance such as collagen etc. and it is possible to carry out mass culture in a status close to a living body.

This cell culture platform 120 with frame is preferable to be used together with a holder 125 shown in Figure 15c, because it seals in the active substance secreted by the cells when culturing within the cell house (cell culture platform). Moreover, the use of the holder 125 improves the culture efficiency of the cell culture platform, which is preferable.

### Embodiment 1

Composition of the liquid to form hydroxyapatite and carbonate apatite-containing hydroxyapatite
Calcium ion (Ca+2):15mM
Phosphoric acid ion (HPO4-2):9mM
Chlorine ion (Cl-):710mM
Hydrogen carbonate ion (HCO3-):21mM
Sodium ion (Na+):730ml

Bubbling by carbon dioxide gas is carried out in the hydroxyapatite formation liquid of the above composition so as to adjust pH to be 6.1 at 37°C.

## Claims

1. A medical material (10), comprising a structure (11) formed from a plurality of jointed porous three dimensional structures with porosity of 20 to 97 %, the structure having a hole and/or a hollow section (12, 13, 14, 15) formed in a void between the porous three dimensional structures, wherein the hole and/or the hollow section (12, 13, 14, 15) is expanded dendritically inside the structure (11), wherein the three dimensional porous structure is composed of one or more materials selected from a group of pure titanium, titanium alloy, cobalt chrome alloy, synthetic polymer, natural polymer, stainless steel, stainless alloy, aluminum and aluminum alloy, and wherein the material has a thin fiber shape part having thickness of 100 *µ*m or less.

2. A medical material according to claim 1, wherein the porosity of the porous three dimensional structure varies gradiently in a direction from a surface to an inside.

3. A medical material according to claim 1 or 2, wherein the porous three dimensional structure has at least one or more detachable rigid bars (66, 67, 68, 69, 70) penetrating the hole and/or the hollow section (12, 13, 14, 15).

4. A medical material according to claim 3, wherein a thickness of the rigid bar (66, 67, 68, 69, 70) is 0.5 square centimeters or less in a cross section area.

5. A medical material according to claim 3 or 4, wherein a cross sectional shape of the rigid bar (66, 67, 68, 69, 70) is any shape selected from a group of round, oval, a modified circular shape with lacking part, polygon, L-shape, H-shape and hollow shape.

6. A medical material according to any one of claims 1 to 5, wherein a substance is diffused and distributed inside the three dimensional porous structure, by injecting the substance into the hole and/or hollow section (12, 13, 14, 15).

7. A medical material according to any one of claims 1 to 6, wherein at least a part of the three dimensional porous structure contains either calcium phosphate or ceramics apatite.

8. A medical material according to any preceding claim, wherein a coating of ceramics apatite is provided on a periphery of the thin fiber shape part composing the three dimensional porous structure.

9. A medical material according to any preceding claim, wherein the three dimensional porous structure is made by laminating a sheet having a thickness of 0.1 to 10 mm and hardening with pressurization or sintering.

10. A medical material according to claim 6, wherein the substance injected into the three dimensional porous structure has a physiological function and is at least one or more selected from the group composed of various cells, bone marrow cells, bone marrow fluid, stem cells separated from bone marrow fluid, umbilical cord blood derived cells, peripheral blood-derived cells, fragmented tissues, various proteins, lipids, polysaccharides, enzymes, antibiotics, antibacterial agents, hormones, cytokines, blood coagulation accelerants, cell growth promoting factors, extracts from genetically modified cells, substances produced from genetically modified cells, vascular endothelial cell growth factors (VEGF), platelet-induced growth factors (PIGF), therapeutic gain factors beta 1 (TGF. beta. 1), acid fibroblasts growth factor (aFGF), basic fibroblasts growth factor (bFGF), transforming factors alpha (TGF. alph), epithelial cell growth factors, osteonectin, antiopoietin (ANG1), ANG2, blood platelet-derived growth factors AA, blood platelet-derived growth factors BB, bone morphogenetic proteins (BMP), hepatic cell growth factors (HGF), extracellular matrices, collagens, or complexes or derivatives of any of them.

## Patentansprüche

1. Medizinisches Material (10), das einen Aufbau (11) umfasst, der aus einer Vielzahl von zusammengefügten porösen dreidimensionalen Strukturen mit einer Porosität von 20 bis 97 % ausgebildet ist, wobei der Aufbau ein Loch und/oder einen hohlen Teilabschnitt (12, 13, 14, 15) hat, das/der in einer Lücke zwischen den porösen dreidimensionalen Strukturen ausgebildet ist, wobei das Loch und/oder der hohle Teilabschnitt (12, 13, 14, 15) innerhalb des Aufbaus (11) dendritisch erweitert ist, wobei sich die dreidimensionale poröse Struktur aus einem Material oder mehreren Materialien zusammensetzt, das/die aus einer Gruppe von reinem Titan, Titanlegierung, Cobalt-Chrom-Legierung, synthetischem Polymer, natürlichem Polymer, nichtrostendem Stahl, nichtrostender Legierung, Aluminium und Aluminiumlegierung ausgewählt ist/sind, und wobei das Material einen Teil in Form einer dünnen Faser hat, der eine Dicke von 100 µm oder weniger hat.

2. Medizinisches Material nach Anspruch 1, wobei die Porosität der porösen dreidimensionalen Struktur gradientenartig in einer Richtung von einer Oberfläche zu einer Innenseite variiert.

3. Medizinisches Material nach Anspruch 1 oder 2, wobei die poröse dreidimensionale Struktur mindestens einen oder mehr lösbare starre Stäbe (66, 67, 68, 69, 70) hat, die das Loch und/oder den hohlen Teilabschnitt (12, 13, 14, 15) durchdringen.

4. Medizinisches Material nach Anspruch 3, wobei eine Dicke des starren Stabs (66, 67, 68, 69, 70) in einer Querschnittsfläche 0,5 Quadratzentimeter oder weniger beträgt.

5. Medizinisches Material nach Anspruch 3 oder 4, wobei eine Querschnittsform des starren Stabs (66, 67, 68, 69, 70) eine beliebige Form ist, die aus einer Gruppe von rund, oval, einer modifizierten Kreisform mit fehlendem Teil, Polygon, L-Form, H-Form und hohler Form ausgewählt ist.

6. Medizinisches Material nach einem der Ansprüche 1 bis 5, wobei innerhalb der dreidimensionalen porösen Struktur eine Substanz durch Injizieren der Substanz in das Loch und/oder den hohlen Teilabschnitt (12, 13, 14, 15) verbreitet und verteilt ist.

7. Medizinisches Material nach einem der Ansprüche 1 bis 6, wobei zumindest ein Teil der dreidimensionalen porösen Struktur entweder Calciumphosphat oder keramisches Apatit enthält.

8. Medizinisches Material nach einem vorstehenden Anspruch, wobei auf einem Umfang des Teils in Form der dünnen Faser, der die dreidimensionale poröse Struktur bildet, eine Beschichtung aus keramischem Apatit vorgesehen ist.

9. Medizinisches Material nach einem vorstehenden Anspruch, wobei die dreidimensionale poröse Struktur durch Schichten einer Lage, die eine Dicke von 0,1 bis 10 mm hat, und Härten mit Druckbeaufschlagung oder Sintern hergestellt ist.

10. Medizinisches Material nach Anspruch 6, wobei die in die dreidimensionale poröse Struktur injizierte Substanz eine physiologische Funktion hat und mindestens eine Substanz oder mehrere Substanzen ist, die aus der Gruppe ausgewählt ist/sind, die aus verschiedenen Zellen, Knochenmarkzellen, Knochenmarksflüssigkeit, von Knochenmarksflüssigkeit getrennten Stammzellen, von Nabelschnurblut abgeleiteten Zellen, von peripherem Blut abgeleiteten Zellen, zerstückelten Geweben, verschiedenen Proteinen, Lipiden, Polysacchariden, Enzymen, Antibiotika, antibakteriellen Mitteln, Hormonen, Zytokinen, Blutgerinnungsbeschleunigern, zellwachstumsfördernden Faktoren, Extrakten aus genetisch modifizierten Zellen, aus genetisch modifizierten Zellen erzeugten Substanzen, Vascular Endothelial Cell Growth Factors (VEGF), Platelet-Induced Growth Factors (PIGF), Therapeutic Gain Factors Beta 1 (TGF. beta. 1), Acid Fibroblasts Growth Factor (aFGF), Basic Fibroblasts Growth Factor (bFGF), Transforming Factors Alpha (TGF. alph), Epithelzellenwachstumsfaktoren, Osteonectin, Angiopoietin (ANG1), ANG2, von Blutplättchen abgeleiteten Wachstumsfaktoren AA, von Blutplättchen abgeleiteten Wachstumsfaktoren BB, Bone Morphogenetic Proteins (BMP), Hepatic Cell Growth Factors (HGF), extrazellulären Matrizen, Kollagenen oder Komplexen oder Derivaten von jedem davon besteht.

## Revendications

1. Matériau médical (10), comprenant une structure (11) formée à partir d'une pluralité de structures tridimensionnelles poreuses réunies ayant une porosité comprise entre 20 et 97 %, la structure ayant un trou et/ou une section creuse (12, 13, 14, 15) formés dans un vide entre les structures tridimensionnelles poreuses, où le trou et/ou la section creuse (12, 13, 14, 15) s'étendent de manière dendritique à l'intérieur de la structure (11), où la structure tridimensionnelle poreuse se compose d'un ou de plusieurs matériaux sélectionnés parmi un groupe constitué du titane pur, d'un alliage de titane, d'un alliage de chrome et de cobalt, d'un polymère de synthèse, d'un polymère naturel, de l'acier inoxydable, d'un alliage inoxydable, de l'aluminium et d'un alliage d'aluminium, et où le matériau présente une partie en forme de fibre mince ayant une épaisseur de 100 µm ou moins.

2. Matériau médical selon la revendication 1, dans lequel la porosité de la structure tridimensionnelle poreuse varie par gradients dans un sens allant d'une surface vers un intérieur.

3. Matériau médical selon la revendication 1 ou 2, dans lequel la structure tridimensionnelle poreuse présente au moins une ou plusieurs barres rigides amovibles (66, 67, 68, 69, 70) pénétrant dans le trou et/ou la section creuse (12, 13, 14, 15).

4. Matériau médical selon la revendication 3, dans lequel une épaisseur de la barre rigide (66, 67, 68, 69, 70) est de 0,5 centimètres carrés ou moins en section transversale.

5. Matériau médical selon la revendication 3 ou 4, dans lequel une forme en section transversale de la barre rigide (66, 67, 68, 69, 70) est une forme quelconque sélectionnée parmi un groupe constitué d'une forme ronde, d'une forme ovale, d'une forme circulaire modifiée avec une partie manquante, d'un polygone, d'une forme en L, d'une forme en H et d'une forme creuse.

6. Matériau médical selon l'une quelconque des revendications 1 à 5, dans lequel une substance est diffusée et distribuée à l'intérieur de la structure tridimensionnelle poreuse, en injectant la substance dans le trou et/ou la section creuse (12, 13, 14, 15).

7. Matériau médical selon l'une quelconque des revendications 1 à 6, dans lequel au moins une partie de la structure tridimensionnelle poreuse contient soit du phosphate de calcium, soit de l'apatite céramique.

8. Matériau médical selon l'une quelconque des revendications précédentes, dans lequel un revêtement d'apatite céramique est prévu sur une périphérie de la partie en forme de fibre mince composant la structure tridimensionnelle poreuse.

9. Matériau médical selon l'une quelconque des revendications précédentes, dans lequel la structure tridimensionnelle poreuse est obtenue par laminage d'une feuille ayant une épaisseur comprise entre 0,1 et 10 mm et durcissement par application de pression ou frittage.

10. Matériau médical selon la revendication 6, dans lequel la substance injectée dans la structure tridimensionnelle poreuse a une fonction physiologique et est au moins un élément ou plusieurs éléments sélectionnés parmi le groupe constitué de diverses cellules, de cellules de moelle osseuse, de fluide de moelle osseuse, de cellules souches extraites du fluide de moelle osseuse, de cellules obtenues à partir de sang de cordon ombilical, de cellules obtenues à partir de sang périphérique, de tissus fragmentés, de diverses protéines, de lipides, de polysaccharides, d'enzymes, d'antibiotiques, d'agents antibactériens, d'hormones, de cytokines, d'accélérateurs de coagulation sanguine, de facteurs favorisant la croissance cellulaire, d'extraits de cellules génétiquement modifiées, de substances produites à partir de cellules génétiquement modifiées, de facteurs de croissance de la vascularisation endothéliale (VEGF), de facteurs de croissance cellulaire induite par les plaquettes (PIGF), de facteurs de gain thérapeutique bêta 1 (TGF. bêta. 1), d'un facteur de croissance des fibroblastes acide (aFGF), d'un facteur de croissance des fibroblastes basique (bFGF), de facteurs de croissance transformants alpha (TGF. alph), de facteurs de croissance de cellules épithéliales, de l'ostéonectine, de l'angiopoiétine (ANG1), de l'ANG2, de facteurs de croissance dérivés des plaquettes sanguines AA, de facteurs de croissance dérivés des plaquettes sanguines BB, de protéines morphogénétiques osseuses (BMP), de facteurs de croissance des hépatocytes (HGF), de matrices extracellulaires, de collagènes, ou de complexes ou dérivés de l'un de ceux-ci.
